# EUROPEAN PATENT SPECIFICATION

(11) **EP 1 125 595 B1**
(45) Date of publication and mention of the grant of the patent: **31.05.2006**
(21) Application number: 01300720.8
(22) Date of filing: 26.01.2001
(51) Int. Cl.: A61M 16/04, A61M 16/06, B29C 41/04

(54) **Method of making a mask**
Verfahren zur Herstellung einer Maske
Procédé de fabrication d' un masque

(30) Priority: 08.02.2000 GB 0002805
(43) Date of publication of application: 22.08.2001
(73) Proprietor: Smiths Group plc, London NW11 8DS (GB)
(72) Inventor: Doane, Mark William, North Fort Myers, Florida 33917 (US); Thomas, James Edward, New Orleans, Louisiana 70183 (US)
(74) Representative: Flint, Jonathan McNeill

(56) References cited:
- EP-A- 0 911 049
- EP-A- 0 935 971
- WO-A-00/61213
- GB-A- 2 038 703
- US-A- 5 355 879

## Description

This invention relates to methods of making masks.

Masks, such as face masks or laryngeal masks, comprise a relatively stiff mount, cone or shoe member and a softer, more flexible annular balloon, cuff or cushion extending around the edge of the mount, which conforms readily to the anatomy and makes sealing contact with the patient tissue. The cuff is formed separately from the mount and is subsequently joined with it, such as by means of an adhesive or solvent. The cuff may be made by an injection moulding or rotational moulding technique; the mount is usually made by an injection moulding technique. Examples of laryngeal masks and their manufacture are shown in US 5305743, US 5297547, US 5282464, GB 2267034, US 5249571, US 5241956, US 5303697, GB 2249959, GB 2111394, EP 448878, US 4995388, GB 2205499, GB 2128561, GB 2298797, GB 2334215, PCT/GB00/03045, PCT/GB00/03044 and GB 2337020. US 5355879 describes various types of laryngeal mask, which may be moulded in one piece or have a separate cuff attached adhesively to a backplate. GB 2038703 describes a face mask having a semi-rigid cone and a foamed cushion bonded to the cone. WO 0061213 describes a laryngeal mask formed by rotational moulding.

Masks made in this way are relatively expensive because of the need for different manufacturing and assembly operations. The join between the mount and cushion provides a possible site for failure or leakage and requires testing to ensure an effective join. Where the mask is used internally, such as in a laryngeal mask, the consequences of separation of the cushion and mount can be severe. The join itself may make the mask stiffer or may make it more difficult to achieve exactly the desired flexibility.

It is an object of the present invention to provide an alternative method of manufacture for a mask.

According to the present invention, there is provided a method of making a mask comprising the steps of preforming a mount member, providing a mould having a first part and a second part, the first part having an aperture for receiving the mount member and the second part having a recess defining the shape of a cuff, adding a fluid plastics material to the recess, placing the preformed mount member in the aperture of the first part, placing the first and second parts of the mould together, rotating the mould so that the fluid plastics material coats the recess to form a thin layer of gelled plastics in the recess, rotating the mould so that the fluid plastics material coats that part of the mount member that covers the recess to bond with the mount member, and removing the mount member from the mould when a hollow cuff has been rotationally moulded thereon.

A laryngeal mask assembly and a face mask assembly according to the present invention, will now be described, by way of example, with reference to the accompanying drawings, in which:
- Figure 1: is a partly-sectional side elevation view of a larnygeal mask assembly for background information;
- Figure 2: is a schematic sectional side elevation view of moulding apparatus used to form the mask of the assembly of Figure 1 at a first stage in the moulding operation;
- Figure 3: is a sectional side elevation view of the moulding apparatus of Figure 2 at a subsequent stage in the moulding operation;
- Figure 4: is a sectional side elevation view of a face mask made according to the method of present invention;
- Figure 5: is a sectional, perspective view of the mould used to make the face mask of Figure 4; and
- Figure 6: shows the underside of the cone of the face mask at a preliminary stage of manufacture.

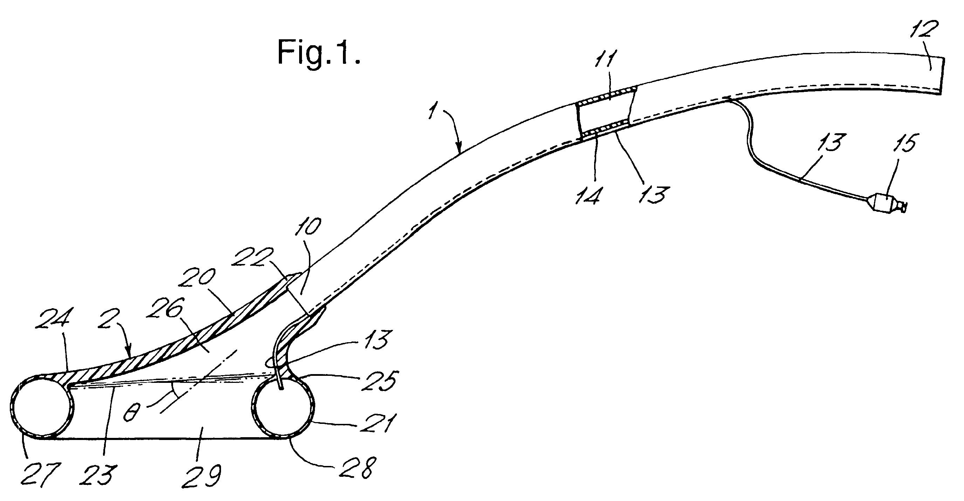

The laryngeal mask shown in Figures 1 to 3 and described below is for background information useful for an understanding of the invention.

With reference first to Figure 1, the laryngeal mask assembly comprises a tube 1 and a mask 2 mounted at the patient end 10 of the tube.

The tube 1 is of a bendable plastics material, such as PVC and is curved along its length. A bore 11 extends along the tube 1 from its patient end 10 to its rear, machine end 12. A small-bore inflation line 13 extends along the length of the tube 1, within a channel 14 formed along the outside of its wall, such as in the manner described in PCT/GB00/03044. Towards its machine end, the inflation line 13 extends away from the tube 1 and is connected to a combined valve and coupling 15 of the usual kind.

The mask 2 comprises a mount 20 and an inflatable cuff 21. The mount 20 is of a plastics material and is generally of a shoe or funnel shape. It has a relatively thick wall so that it is relatively stiff. The rear, machine end of the mount 20 has a neck 22 of circular section embracing and bonded to the patient end 10 of the tube 1. A silicone gasket (not shown) may be inserted between the tube 1 and the mount 20 to improve the seal. The mount 20 tapers outwardly from the machine end 22 to its patient end 23, which is inclined to the axis of the machine end at an angle θ of about 25° so that the patient end of the mount has an oval shape with its forward end 24 being more pointed than its rear end 25. The patient end 23 of the mount 20 is inclined to face towards the inner side of the curve of the tube 1. Internally, the machine end 22 of the mount 20 communicates with a cavity 26 in the mount that increases in cross-sectional area along its length, from the machine end.

The cuff 21 is formed integrally as a single piece with the mount 20 and is of the same plastics material but has a thinner wall so that it is softer and more flexible. The cuff 21 is formed into an annulus of the same shape as the patient end 23 of the mount 20 and is oval with its forwardly-directed end 27 being more pointed than its rearwardly-directed end 28. The cuff 21 encloses a central region 29 of the same shape as the patient end 23 of the mount 20. The inflation line 13 extends beyond the patient end 10 of the tube, is moulded into the mount 20 and projects into the cuff 21 so that the cuff can be inflated and deflated via the inflation line. When inflated in position in a patient, the cuff 21 expands to contact patient tissue in the region of the hypopharnyx.

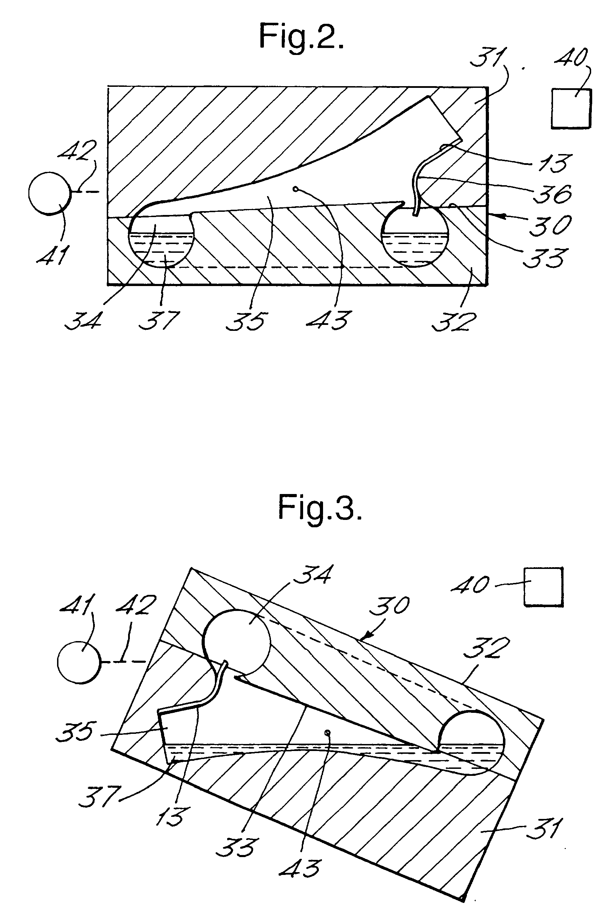

The cuff 21 and mount 20 are formed using rotational moulding apparatus shown in Figures 2 and 3. The apparatus includes a mould 30 in two parts: an upper part 31 and a lower part 32, which can be separated after use to enable the component to be removed. Around the upper surface 33 of the lower part 32 extends an annular channel 34 the wall of which corresponds to the external shape of the cuff 21. The lower surface of the upper part 31 of the mould has a recess 35 of approximately funnel shape, the wall of which corresponds to the external shape of the mount 20. Additionally, the upper part 31 has a groove 36 in which is clipped the patient end of the inflation line 13. The inflation line 13 projects a short distance beyond the patient end of the groove 36, into the channel 34. A PTFE-coated wire, or a solid PTFE rod (not shown) is inserted in the line 13 during moulding; to prevent occlusion, and is subsequently removed. The moulding apparatus additionally includes a conventional heater 40 and displacement means 41 for altering the orientation of the mould 30 as desired.

Initially, the mould 30 is oriented as shown in Figure 2, with the upper part 31 uppermost. A measured volume of plastisol 37, or other heat-gellable plastics in fluid form, is added to the mould 30 through an inlet passage, not shown, so that the plastics flows into the channel 34 in the lower part 32. The plastics is preferably in liquid form but could be in other fluid form, such as a powder. The mould 30 is then rocked about the x-axis 42 and the z-axis 43 so that the plastisol comes into contact with the entire surface of the channel 34. The mould 30 is heated by the heater 40 so that the plastisol 37 gels where it contacts the surface of the mould. This rocking movement is relatively brief so that the layer of plastics material built up on the surface of the channel 34 is only thin and, in particular, is of the appropriate thickness to provide the necessary flexibility of the cuff 21. It can be seen that the plastics deposited on the surface of the channel 34 provides a hollow, tubular formation of annular shape.

After the desired thickness of plastics has been laid down on the surface of the channel 34, the moulding apparatus moves to a second phase in which the mould 30 is rotated about the z-axis 43 as shown in Figure 3. When this happens, the remaining plastisol 37 runs out of the channel 34 and into one side of the funnel-shape recess 35. The quantity of plastisol deposited on the wall of the channel 34 is small compared with the total volume, so the majority of the plastisol is still liquid in the second phase. The mould 30 is gradually rotated and rocked about the x-axis 42 and the z-axis 43 so that the plastisol 37 is deposited on the wall of the recess 35. This process takes longer than that used to form the cuff 21, because the desired wall thickness of the mount portion 20 is substantially greater than that of the cuff. Typically, the wall of the mount 20 would have a thickness of several millimetres, whereas the wall of the cuff 21 would only be a fraction of a millimetre. The quantity of plastisol used is preferably such that when the desired wall thickness of the mount 20 has been deposited, all the plastisol has been gelled. During either of these rotational moulding movements it may be necessary also to rotate or rock the mould 30 about the y-axis. The inflation line 13 extending along the groove 36 becomes embedded within the thickness of the wall of deposited plastics material. It may be necessary to block the patient end of the inflation line 13 with a removable insert, such as a wire, in order to prevent it becoming blocked by the plastics material. Instead of moulding the mask about the inflation line, the mask could be moulded with a small bore, such as by means of a wire core pin within the mould. One end of the bore would extend to the machine end of the mask and the other end would open inside the cuff. The bore could make connection with an inflation line extending within the wall of the tube.

When all the plastisol has gelled, the mould 30 is heat treated in the usual way fully to cure the plastics. The mould 30 is then separated into its two parts 31 and 32 and the mask 2 is removed. After removal of any sprue or excess plastic, the mask 2 is joined to the tube 1 in the usual way, with the inflation line 13 being clipped into the channel 14. The cuff 21 can then be inflated or deflated as desired via the inflation line 13.

In practice, the moulding apparatus would have several moulds mounted on a conveyor, turntable or the like, which pass through various stations at which the plastisol is added, the mould is oriented as appropriate, the mould is heat treated, and the finished component is removed.

The cuff and mount described above are formed integrally.

The present invention realises that it is not essential for the mount and cuff to be formed integrally since advantages can also be achieved where the mount is pre-formed and the cuff is subsequently moulded with the mount. This still achieves simplification of manufacture and enhanced integrity of the join between cuff and mount. If the mount or cone needs to be clear and transparent, such as in face masks where it is important to be able to see the patient's mouth region, rotational moulding may not be suitable because this technique cannot yet produce the necessary transparency. Rotational moulding is still, however, a desirable technique for forming the cuff of the mask. Whereas in previous face masks, where both the mount/cone and cuff are preformed and subsequently bonded together, the present invention is to mould the cuff directly onto the pre-formed mount/cone so that the moulding operation itself produces the bond between the cuff and the cone.

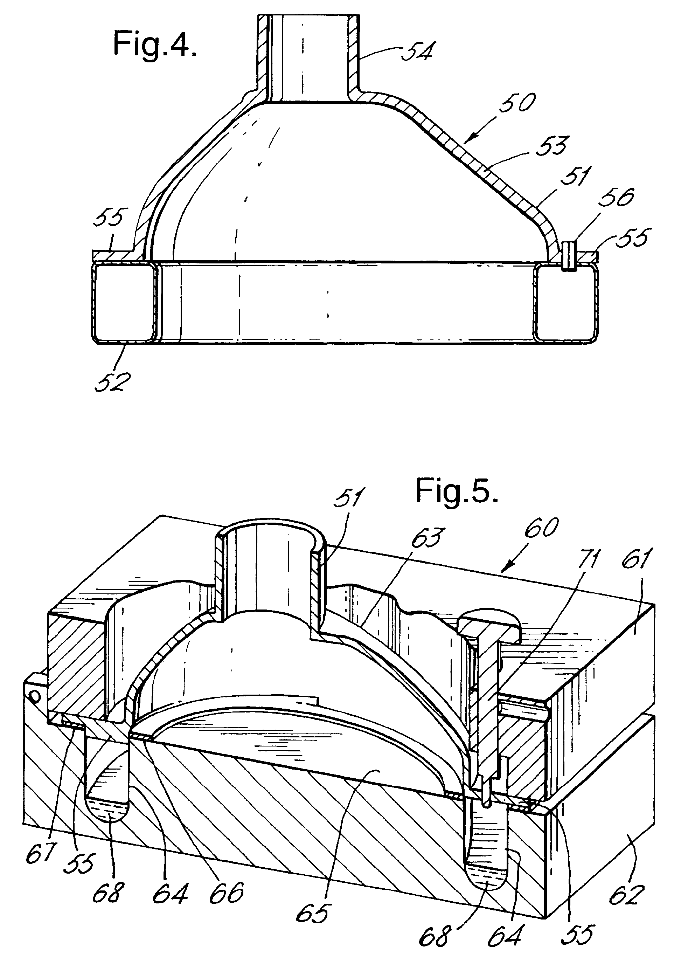

With reference to Figure 4, there is shown a face mask 50 comprising an upper mount member or cone 51 and a lower cuff 52. The cone 51 has a main body 53 of domed shape with a short tubular connector 54 at its upper end and a narrow outwardly-projecting flange 55 at its lower end, which is oval in shape. The cone 51 is of a clear, transparent plastics material, typically PVC, and is made by an injection-moulding technique, or by some other technique that achieves the necessary transparency. The cuff 52 is a hollow, inflatable tubular balloon having an inflation inlet 56 extending through the flange 55 of the cone 51. The cuff 52 is shaped to seal around the nose and mouth.

With reference now also to Figure 5, the pre-formed cone 51 is placed in a rotational mould 60 comprising an upper part 61 of a heat-insulating material, such as PTFE, and a lower part 62 of a metal, such as aluminium. The upper part 61 has a large aperture 63 in which the main part of the cone 51 is located, there being a clearance between the outside of the cone and the inside of the aperture. The lower part 62 of the mould has an annular recess 64 in its upper surface 65 of the same oval shape as the lower edge of the cone 51. In section, the recess 64 defines the shape of the uninflated cuff 52. Two insulating gaskets 66 and 67 of a low durometer silicone are secured to the upper surface of the mould part 62, around the inside and outside respectively of the recess 64.

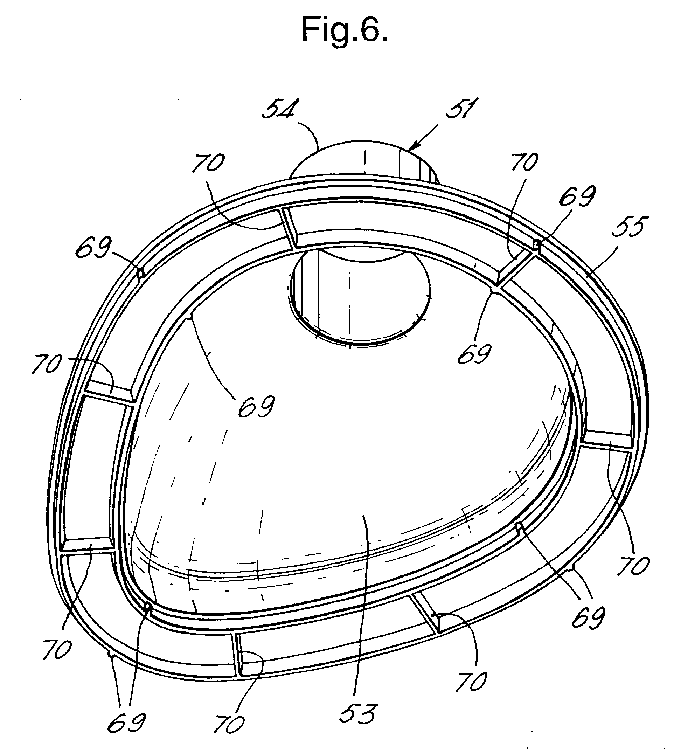

Initially, a measured quantity of plastisol 68, or similar material is poured in the recess 64 in the lower part 62 of the mould 60. The cone 51 is then placed on the lower part 62 of the mould with the flange 55 extending around the opening of the recess 64 and sitting on the inner and outer gaskets 66 and 67. The lower surface of the flange 55 is shown in Figure 6, where it can be seen that it has positioning tabs 69 and ribs 70 that engage in corresponding recesses (not shown) in the upper surface 65 of the lower part 62 of the mould 60. The upper part 61 of the mould 60 is then hinged down so as to trap the flange 55 between the two parts. A pin 71 mounted in the upper part 61 of the mould 60 projects downwardly through a hole in the flange 55 a short distance into the recess 64. The entire mould 60 is then rotated in two directions so as to coat the surface of the recess 64 with the plastisol 68. The lower part 62 of the mould 60 is heated, such as with infra-red lamps, so as to gel the plastisol coated on the surface of the recess 64. The mould 60 is rotated in two directions and stops with the upper part 61 below the lower part 62. In this position, the plastisol 68 flows towards the cone flange 55 where it melts the ribs 70 and the lower surface of the flange, to bond securely with it. After appropriate curing, the two parts 61 and 62 of the mould are separated and the completed face mask 50 is removed. The inflation inlet 56 is subsequently inserted into and sealed with the hole made by the pin 71. This technique enables a hollow cuff to be made by rotational moulding and a cone to be made by a different method whilst avoiding manual assembly operations and ensuring a secure bond.

It will be appreciated that the cuff of a face mask, laryngeal mask or the like need not necessarily be hollow but could be of a foam.

## Claims

1. A method of making a mask comprising the steps of preforming a mount member (51), providing a mould (60) having a first part (61) and a second part (62), the first part having an aperture for receiving the mount member (51) and the second part (62) having a recess (64) defining the shape of a cuff (52), adding a fluid plastics material (68) to the recess (64), placing the preformed mount member (51) in the aperture of the first part (61), placing the first and second parts (61 and 62) of the mould together, rotating the mould (60) so that the fluid plastics material (68) coats the recess (64) to form a thin layer of gelled plastics in the recess, rotating the mould (60) so that the fluid plastics material (68) coats that part of the mount member (51) that covers the recess to bond with the mount member, and removing the mount member (51) from the mould (60) when a hollow cuff (52) has been rotationally moulded thereon.

## Patentansprüche

1. Verfahren zur Herstellung einer Maske mit den Schritten des Vorformens eines Halterungselements (51), des Vorsehens einer Form (60) mit einem ersten Teil (61) und einem zweiten Teil (62), wobei der erste Teil eine Öffnung zum Aufnehmen des Halterungselements (51) aufweist und der zweite Teil (62) eine Aussparung (64) aufweist, die die Form einer Manschette (52) festlegt, des Zugebens eines flüssigen Kunststoffmaterials (68) in die Aussparung (64), des Anordnens des vorgeformten Halterungselements (51) in der Öffnung des ersten Teils (61), des Anordnens des ersten und des zweiten Teils (61 und 62) der Form zusammen, des Drehens der Form (60), so dass das flüssige Kunststoffmaterial (68) die Aussparung (64) überzieht, um eine dünne Schicht aus geliertem Kunststoff in der Aussparung auszubilden, des Drehens der Form (60), so dass das flüssige Kunststoffmaterial (68) denjenigen Teil des Halterungselements (51) überzieht, der die Aussparung bedeckt, um es mit dem Halterungselement zu verbinden, und des Entnehmens des Halterungselements (51) aus der Form (60), wenn eine hohle Manschette (52) durch Drehen an diesem geformt wurde.

## Revendications

1. Procédé de fabrication d'un masque comprenant les étapes de préformage d'une monture (51), fourniture d'un moule (60) comprenant une première partie (61) et une seconde partie (62), la première partie ayant une ouverture pour recevoir la monture (51) et la seconde partie (62) ayant une cavité (64) définissant la forme d'une manchette (52), ajout d'un matériau plastique fluide (68) dans la cavité (64), mise en place de la monture (51) préformée dans l'ouverture de la première partie (61), mise en place ensemble des première et seconde parties (61 et 62) du moule, rotation du moule (60) afin que le matériau plastique fluide (68) revête la cavité (64) pour former une fine couche de plastique solidifié dans la cavité, rotation du moule (60) afin que le matériau plastique fluide (68) revête la partie de la monture (51) qui couvre la cavité pour la lier avec la monture, et retrait de la monture (51) du moule (60) lorsqu'une manchette creuse (52) a été moulée dessus par rotation.
